# EUROPÄISCHE PATENTSCHRIFT

(11) **EP 0 743 049 B1**
(45) Veröffentlichungstag und Bekanntmachung des Hinweises auf die Patenterteilung: **29.08.2001**
(21) Anmeldenummer: 95810329.3
(22) Anmeldetag: 18.05.1995
(51) Int. Cl.: A61F 2/34, A61F 2/46

(54) **Künstliche Gelenkschale**
Artificial acetabular cup
Cupule acétabulaire artificielle

(43) Veröffentlichungstag der Anmeldung: 20.11.1996
(73) Patentinhaber: Sulzer Orthopädie AG, 6340 Baar (CH)
(72) Erfinder: Limacher, Urs, CH-6331 Hünenberg (CH); Lamprecht, Stefan, CH-8309 Birchwil (CH)
(74) Vertreter: Sulzer Management AG

(56) Entgegenhaltungen:
- EP-A- 0 649 641
- DE-A- 4 429 026

## Beschreibung

Die Erfindung handelt von einer künstlichen Gelenkschale mit einer Aussenschale und mit einer Innenschale, die mit der Aussenschale eine gemeinsame Trennfläche aufweist und die in der Aussenschale mit einer Klemmverbindung verankerbar ist, wobei die Klemmverbindung durch Reibschluss zwischen Innenschale und Aussenschale gebildet ist.

Die Erfindung handelt ferner von einer Ausziehhilfe zum Ausziehen einer Innenschale aus einer Anssenschale gemäß Anspruch 5.

Doppelte künstliche Gelenkschalen mit einer Schnappverbindung zwischen der äusseren und der inneren Gelenkschale sind seit vielen Jahren in Gebrauch. Gerade bei Kunststoffinnenschalen macht man von der geringeren Steifigkeit des Kunststoffs Gebrauch, um für Schnappverbindungen einen Formschluss zu erreichen. So zeigt die Patentschrift EP 0 245 527 eine mit Reibung und Formschluss hergestellte Schnappverbindung einer Kunststofflagerschale in einer metallischen Aussenschale. Wegen der geringeren Steifigkeit des Kunststoffs wirken sich Schwankungen in den Herstellmassen bei der Paarung der beiden Teile nur geringfügig auf die Haltekräfte zwischen den beiden Schalen aus. Ausserdem können bei vielen Systemen die Kunststoffinnenschalen nur entfernt werden, wenn die Kunststoffinnenschale oder ein Zwischenglied zerstört werden. Schwieriger wird die Situation, wenn Innen- und Aussenschale aus Metall sind, da die Herstelltoleranzen für die Paarung in der Klemmverbindung wesentlich geringer sein müssen. Im weiteren muss bedacht werden, dass die Ausziehkräfte zum Entfernen einer Innenschale aus einer implantierten Aussenschale nicht so gross sein dürfen, dass der Sitz der implantierten Aussenschale im Knochen gefährdet wird. Abdrückschrauben zum Ausziehen oder Abdrücken der Innenschale sind nur dann möglich, wenn für das Ausziehen das Material der Innenschale und ihre Wandstärke das Anbringen von Gewindebohrungen gestatten, oder wenn für das Abdrücken die Innenschale mit einem Flansch mit Gewindebohrungen versehen ist. Gerade diese Bedingungen sind bei verschleissfesten Lagermaterialien wie z.B. Keramik oder harten Metallegierungen kaum erreichbar.

In der EP-A-0 649 641 ist eine Gleitschale aus Keramik mit einem bestimmten Konuswinkel α in einer Metallschale verankert, um die Auspresskräfte niedrig zu halten. Die Kuppel der Keramikschale liegt hohl in der Kuppel der Metallschale, um die Konusverbindung nicht zu beeinflussen. Im Bereich der möglichen Konusklemmung sind zwei diametral gegenüberliegende Ausnehmungen in der Metallschale angebracht. Zum Heraushebeln der Gleitschale kann ein Ausdrückwerkzeug mit der Form eines Golfschlägers in einer der Ausnehmungen angesetzt werden. Diese Einrichtung hat den Nachteil, dass beim Heraushebeln eine einseitige Belastung am Rand einer implantierten Aussenschale auftreten kann, und dass eine punktförmige Belastungsspitze an der konischen Aussenfläche der Gleitschale entsteht, die zu keramischen Abplatzern innerhalb des Operationsfeldes führen kann.

Es ist daher Aufgabe der Erfindung ein System zu schaffen, das für verschiedene Materialkombinationen von Aussenschale und Innenschale ein einfaches und schonendes Lösen ihrer Verbindung gestattet. Diese Aufgabe wird mit den Kennzeichen vom unabhängigen Anspruch 1 gelöst.

Eine solche Anordnung hat den Vorteil, dass die Verbindung von Innenschale zu Aussenschale durch einen leichten Schlag beim Zusammenfügen herstellbar ist und dass auch bei grossen Haltekräften zwischen den Schalen genügend Fläche durch den Tunnel vorhanden ist, die zu einer gleichmässigen Belastung der Innenschale entlang des Bogens beim Ausziehen führt. Somit können mit einem einzigen Konuswinkel für die selbsthemmende Verbindung Innenschalen aus unterschiedlichen Materialien und mit unterschiedlichen Ausziehkräften in einer Aussenschale angewendet werden.

Für schwer bearbeitbare oder dünnwandige Innenschalen kann die Rinne, die den Tunnel bildet in die Aussenschale verlegt werden. Bei dickwandigen Innenschalen kann die Rinne an der Innenschale angebracht werden.

Dadurch, dass für das Ausziehen der Innenschale an dieser nur gleichmässig verteilte Druckkräfte auftreten, können auch spröde Werkstoffe für die Innenschalen verwendet werden. Im weiteren sind flanschlose und auf der Aussenseite rotationssymmetrische Innenschalen auf diese Weise verwendbar, was Fertigungsvorteile bei schwer bearbeitbaren Werkstoffen bringt. Als Ausziehhilfen eignen sich Zangen, die sich zur Verkürzung des im Tunnel liegenden Drahtbogens auf der Aussenschale abstützen. Ausziehkraft und Abstützkraft sind auf diese Weise über die Innenschale und die Ausziehvorrichtung kurzgeschlossen. Wenn eine an der Ausziehvorrichtung angreifende und nicht kompensierte restliche Einzelkraft notwendig ist, um eine Verkürzung des Drahtbogens vorzunehmen, kann diese Kraft, welche schlussendlich an der implantierten Aussenschale angreift, klein gehalten werden, sofern die mit der Kraft verbundene Bewegung kinematisch untersetzt ist.

Die konische selbsthemmende Verbindung hat weiterhin den Vorteil, dass Innenschalen mit einseitig überhöhtem Rand oder mit einer sonstigen Asymmetrie in einem beliebigen Drehwinkel in einer implantierten Aussenschale eingesetzt werden können und durch das relativ einfache Ausziehen in ihrem Drehwinkel korrigiert werden können.

Im folgenden wird die Erfindung anhand von Ausführungsbeispielen beschrieben. Es zeigen:
- Fig. 1: schematisch eine Explosionszeichnung einer Innenschale mit Rinne, einen in dieser Rinne in einem Bogen durchgesteckten Ausziehdraht und eine entsprechende Aussenschale;
- Fig. 2: schematisch die Elemente von Figur 1 als Zusammenstellung;
- Fig. 3: schematisch ein Beispiel für eine Ausziehvorrichtung, die an einer implantierten Aussenschale angesetzt ist, um eine eingesetzte Innenschale auszuziehen;
- Fig. 4: schematisch einen Schnitt IV in Fig. 3;
- Fig. 5: schematisch einen Schnitt V in Fig. 3;
- Fig. 6: schematisch einen vergrösserten Ausschnitt aus Fig. 4;
- Fig. 7: schematisch einen vergrösserten Schnitt in Fig. 6;
- Fig. 8: schematisch eine Ausziehvorrichtung mit einer Schraube zur Erzeugung der Ausziehkraft.

Die Figuren zeigen eine künstliche Gelenkschale mit einer in einem Knochenbett implantierbaren Aussenschale und einer nachträglich einsetzbaren Innenschale, die über eine reibschlüssige Klemmkraft, vorzugsweise durch eine selbsthemmende konische Verbindung, zueinander fixierbar sind. In der Trennfläche zwischen den beiden Schalen wird ein Tunnel gebildet, welcher in einem Bogen zum Schalengrund hin zwei Punkte am Schalenrand verbindet. Durch den Tunnel kann ein Draht gesteckt werden, um die Innenschale ohne Verletzung mit einer gleichmässig über den Bogen verteilten Ausziehkraft herauszuziehen.

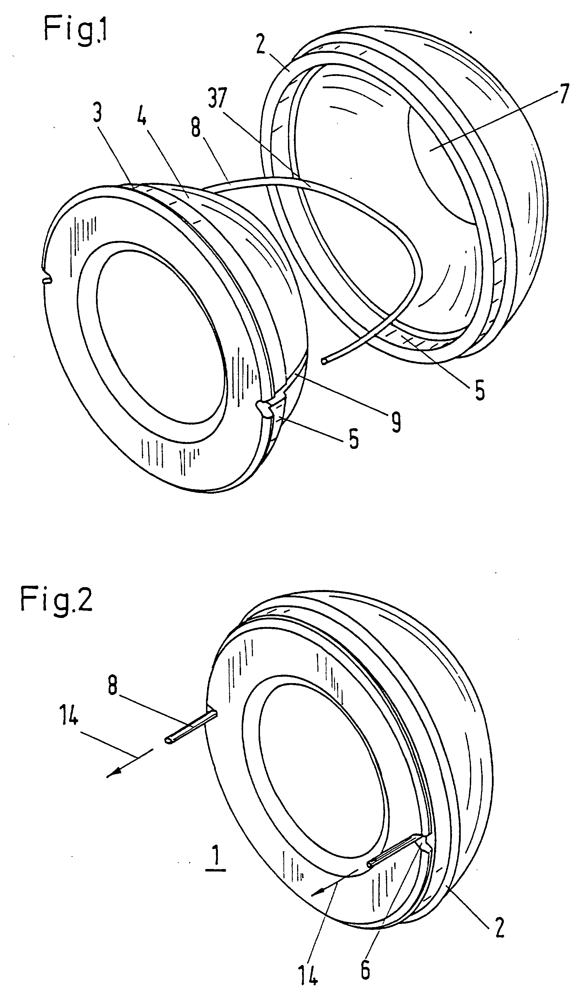

In Figur 1 und 2 ist eine Innenschale 3 über eine selbsthemmende konische Verbindung 5 mit einer Aussenschale 2 verbindbar. In der Trennfläche 4 zwischen den Schalen 2, 3 wird im montierten Zustand ein Tunnel 6 erzeugt, der in einem Bogen 37 zum Schalengrund 7 hin zwei Punkte am Schalenrand verbindet. Der Tunnel 6 wird in diesem Fall durch eine Rinne 9 in der Aussenfläche der Innenschale 3 und durch die Trennfläche 4 gebildet; er kann ebenso durch eine Rinne in der Innenfläche der Aussenschale 2 und durch die Trennfläche 4 an der Innenschale 3 gebildet werden. Der Draht 8 ist elastisch und biegbar so wie ein Klaviersaitendraht oder der mehradrige Zugdraht bei einem Bowdenzug. Wenn am Draht 8 relativ zur Aussenschale 2 in Ausziehrichtung 14 Zug erzeugt wird, verteilt sich die Ausziehkraft über den Bogen 37 an der Innenschale. Zug kann mit festen Ausziehvorrichtungen, die sich auf der Aussenschale abstützen, erzeugt werden oder auch mit einem flexiblen Bowdenzug dessen Führungsmantel sich auf der Aussenschale abstützt.

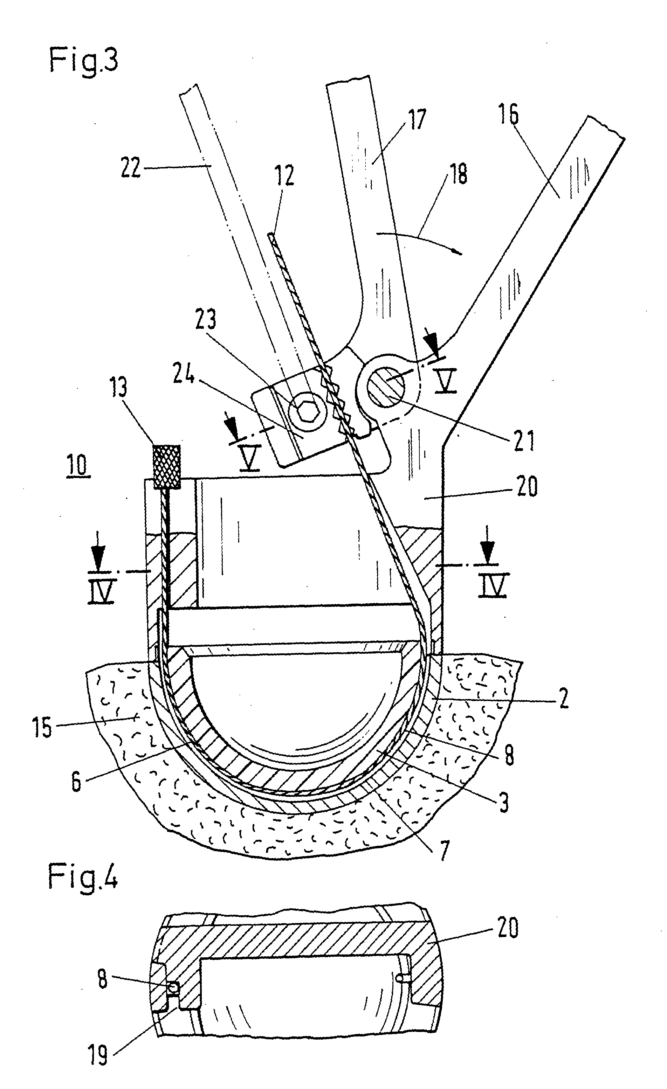

In den Figuren 3 bis 7 ist das Prinzip einer festen Ausziehvorrichtung 10 gezeigt, die einen Aufsetzkörper 20 besitzt, der an beiden Tunnelenden auf der Aussenschale 2 mit einem Fuss 25 aufsitzt. Der Draht 8 ist auf der einen Seite mit einem Kopf 13 im Aufsetzkörper 20 verankert, durch den Tunnel 6 der Gelenkschale geführt und auf der anderen Seite in einem um einen Lagerzapfen 21 schwenkbaren Klemmhebel 17 gehalten.

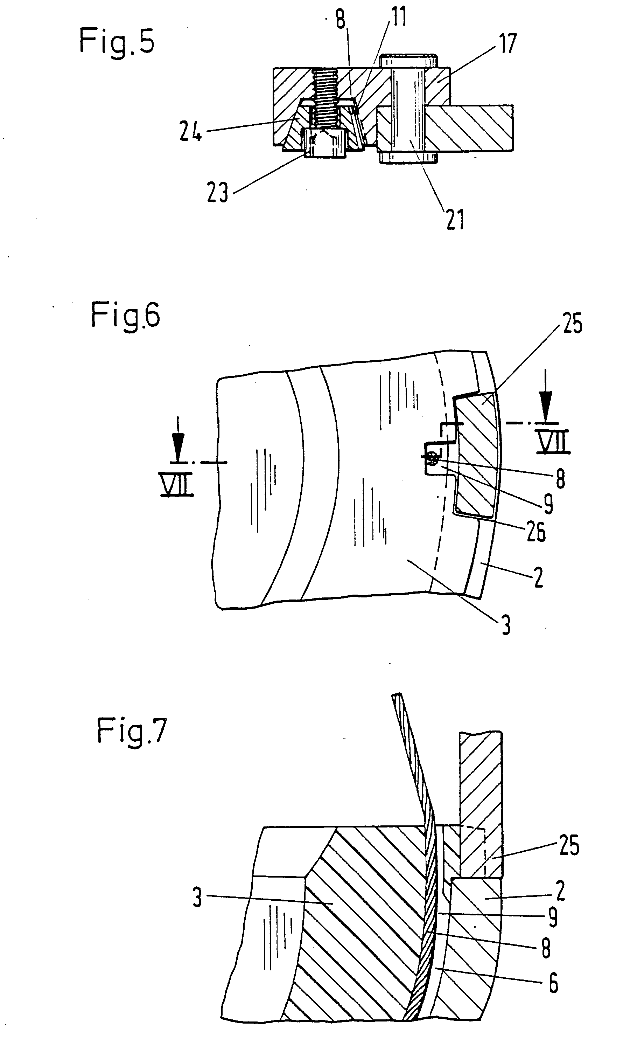

Die Klemmstelle 11 ist leicht gewellt. Sie entsteht durch das Festziehen eines konischen Klemmstücks 24 mit einer Klemmschraube 23 mit Hilfe eines angedeuteten Steckschlüssels 22. Dadurch, dass die Klemmstelle 11 einen kürzeren Abstand zum Lagerzapfen 21 aufweist als die Kraftangriffspunkte an den wesentlich längeren Hebeln 16, 17, wird die Schwenkbewegung in einer Schwenkrichtung 18 untersetzt und eine geringere Kraft notwendig. Bei einem gleichzeitigen manuellen Kraftangriff an den Hebeln 16, 17 kompensieren sich die Kräfte weitgehend und es entstehen kaum zusätzliche Kräfte zwischen der Aussenschale 2 und einem sie umgebenden Knochenbett. Um die Handhabung für das Einführen des Drahtes 8 zu erleichtern, ist dieser mit einer Spitze 12 versehen, die zunächst ohne den Aufsetzkörper 20 das Einfädeln des Drahtes 8 im Tunnel 6 ermöglicht. Die Aufsetzvorrichtung 20 besitzt seitliche Schlitze 19 oder Aussparungen und eine seitlich offene Klemmstelle 11, um den bereits eingefädelten Draht 8 seitlich in die Ausziehvorrichtung einzuführen. In Figur 6 ist der Rand der Innenschale 3 mit einer Aussparung 26 im Bereich der Rinne 9 versehen, um den Fuss 25 der Ausziehvorrichtung auf dem Rand der Aussenschale 2 zu zentrieren.

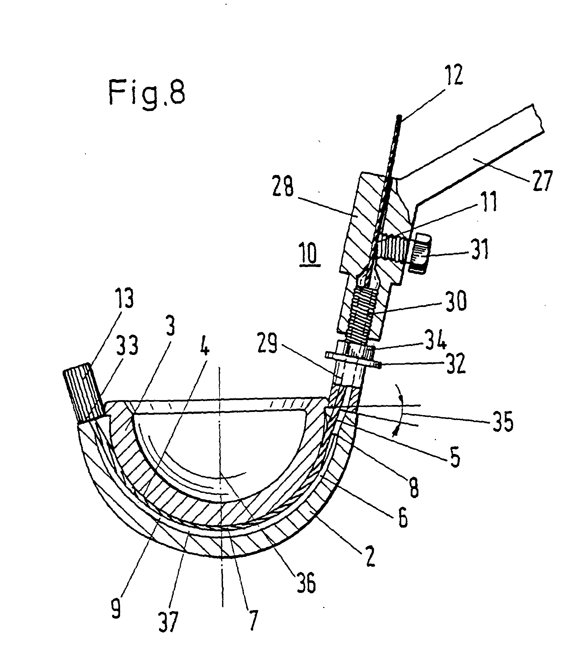

Eine weitere Möglichkeit für eine Ausziehvorrichtung 10 ist in Figur 8 gezeigt. Eine Rinne 9 befindet sich in der Aussenschale 2 und bildet mit der glatten Innenschale 3 einen Tunnel 9. Ein Draht 8 mit Spitze 12 und Kopf 13 wird direkt eingefädelt. Dabei bildet eine Anschrägung 33 mit Winkel 35 am Rand der Aussenschale 2 ein Widerlager für den Kopf 13 und für einen Unterteil 29 in Form einer Hohlschraube, welche über ein Gewinde 30 in einem Oberteil 28 der Ausziehvorrichtung 10 eingeschraubt ist. Ober- und Unterteil 28, 29 werden über den eingezogenen Draht 8 gestülpt und dieser wird mit einer Klemmschraube 31 an einer Klemmstelle 11 geklemmt. Mit einem nicht gezeigten Gabelschlüssel, der auf einer Schulter 32 aufliegt und an Angriffsflächen 32 angreift wird nun der Unterteil 29 herausgeschraubt und gleichzeitig Zug am Draht erzeugt, um die Klemmverbindung 5 zwischen Innen- und Aussenschale zu lösen. Ein Haltegriff 27 am Oberteil 28 sorgt dafür, dass zu dem Moment vom nicht gezeigten Gabelschlüssel ein Gegenmoment erzeugt wird. Wegen der nicht einfachen Platzverhältnisse kann statt dem Ober- und Unterteil 28, 29 auch ein Bowdenzug verwendet werden, dessen Mantelstück analog zum Unterteil 28 an der Aussenschale aufliegt. Der Draht 8 ist dann entsprechend länger ausgeführt.

Ganz generell ist zu bemerken, dass der Bogen 37 nicht durch die Polachse 36 der Innenschale 3 gelegt werden muss, um ein Lösen der Klemmverbindung 5 zu bewirken.

## Patentansprüche

1. Künstliche Gelenkschale (1) mit einer Aussenschale (2) und mit einer Innenschale (3), die mit der Aussenschale eine gemeinsame Trennfläche (4) aufweist und die in der Aussenschale mit einer Klemmverbindung (5) verankerbar ist, wobei die Klemmverbindung durch Reibschluss zwischen Innenschale (3) und Aussenschale (2) gebildet ist, **dadurch gekennzeichnet**, dass die Innenschale (3) oder die Aussenschale (2) eine Rinne (9) aufweist, welche in der Trennfläche (4) mit der Gegenseite einen Tunnel (6) bildet und in einem Bogen (37) zum Schalengrund (7) hin von einem Punkt am Schalenrand zu einem anderen Punkt am Schalenrand führt, wobei durch den Tunnel (6) ein Draht (8) durchsteckbar und mit dem durchgesteckten Draht (8) die Innenschale (3) aus der Aussenschale (2) her ausziehbar ist.

2. Künstliche Gelenkschale nach Anspruch 1, **dadurch gekennzeichnet**, dass der Reibschluss durch eine selbsthemmende konische Verbindung gebildet ist.

3. Künstliche Gelenkschale nach einem der Ansprüche 1 oder 2, **dadurch gekennzeichnet**, dass die Innenschale aus Metall ist.

4. Künstliche Gelenkschale nach einem der Ansprüche 1 oder 2, **dadurch gekennzeichnet**, dass die Innenschale aus Keramik ist.

5. Ausziehhilfe (10) zum Ausziehen einer Innenschale (3) aus einer Aussenschale (2) bei einer Gelenkschale nach einem der Ansprüche 1 bis 4, wobei die Ausziehhilfe einen mit einem Kopf (13) versehenen Draht (8) und einen Fuss (25,29) aufweist und mit dem Fuss (25,29) auf dem Rand der Aussenschale abstützbar ist zur Erzeugung einer Ausziehkraft an dem durch den Tunnel (6) durch steckbaren Draht (8).

6. Ausziehhilfe nach Anspruch 5, **dadurch gekennzeichnet**, dass sie Mittel (13, 23, 24, 31) aufweist, mit denen der durch den Tunnel (6) durchgesteckte Draht in der Ausziehhilfe klemmbar ist, und dass sie weitere Mittel (17, 21, 16; 29, 30, 28) aufweist, mit denen die Drahtlänge des durch den Tunnel (6) durchgesteckten Drahts (8) verkürzbar ist.

7. Ausziehhilfe nach Anspruch 6, **dadurch gekennzeichnet**, dass die Drahtlänge innerhalb der Ausziehhilfe (10) durch die Bewegung einer Klemmstelle (11) verkürzbar ist, wobei die Bewegung der Klemmstelle innerhalb der Ausziehhilfe kinematisch untersetzt ist, um grosse äussere Kräfte beim Betätigen der Ausziehhilfe zu vermeiden.

## Claims

1. Artificial joint shell (1) having an outer shell (2) and an inner shell (3) , the inner shell having a partition surface (4) common to the outer shell and anchorable in the outer shell with a clamped connection (5), with the clamped connection being formed by friction locking between the inner shell (3) and the outer shell (2), **characterised in that** the inner shell (3) or the outer shell (2) has a channel (9) which forms a tunnel (6) with the opposite side in the partition surface (4) and which runs in an arc towards the shell base (7) from a point on the shell edge to another point on the shell edge in a direction, with a wire (8) being able to be passed through the tunnel (6) and with the inner shell (3) being able to be extracted from the outer shell (2) using the inserted wire (8).

2. Artificial joint shell in accordance with claim 1, **characterised in that** the friction locking is formed by a self-locking, conical connection.

3. Artificial joint shell in accordance with claim 1 or claim 2, **characterised in that** the inner shell is of metal.

4. Artificial joint shell in accordance with claim 1 or claim 2, **characterised in that** the inner shell is of ceramic material.

5. Extraction tool (10) for pulling an inner shell (3) out of an outer shell (2) in a joint shell in accordance with one of the claims 1 to 4, with the extraction tool having a wire (8) provided with a head (13) and a foot (25, 29) and being supportable on the edge of the outer shell in order to generate an extraction force on the wire (8) which can be passed through the tunnel (6).

6. Extraction tool in accordance with claim 5, **characterised in that** it has means (13, 23, 24, 31) with which the wire passed through the tunnel (6) can be clamped in the extraction tool and in that it has further means (17, 21 16; 29, 30, 28) with which the wire length of the wire (8) passed through the tunnel (8) can be shortened.

7. Extraction tool in accordance with claim 6, **characterised in that** the wire length inside the extraction tool (10) can be shortened by the movement of a clamping point (11), with the movement of the clamping point being geared down kinematically in order to avoid large external forces on actuation of the extraction tool.

## Revendications

1. Cupule acétabulaire artificielle (1) avec une coque externe (2) et une coque interne (3) qui présente avec la coque externe une face de séparation commune (4) et qui peut être ancrée dans la coque externe par une liaison à serrage (5), où la liaison à serrage est formée par une liaison à friction entre la coque interne (3) et la coque externe (2), **caractérisée en ce que** la coque interne (3) ou la coque externe (2) présente une rainure (9) qui forme dans la face de séparation (4) avec le côté opposé un tunnel (6) et qui mène selon un arc (37) vers le fond de la coque (7) d'un point au bord de la coque à un autre point au bord de la coque, où un fil (8) peut être passé à travers le tunnel (6), et la coque interne (3) peut être retirée de la coque externe (2) avec le fil passé (8).

2. Cupule acétabulaire artificielle selon la revendication 1, **caractérisée en ce que** la liaison à friction est formée par une liaison conique autobloquante.

3. Cupule acétabulaire artificielle selon l'une des revendications 1 ou 2, **caractérisée en ce que** la coque interne est en métal.

4. Cupule acétabulaire artificielle selon l'une des revendications 1 ou 2, **caractérisée en ce que** la coque interne est en céramique.

5. Aide à l'extraction (10) pour extraire une coque interne (3) d'une coque externe (2) dans une cupule acétabulaire selon l'une des revendications 1 à 4, où l'aide à l'extraction présente un fil (8) muni d'une tête (13) ainsi qu'une base (25, 29) et peut prendre appui avec la base (25, 29) sur le bord de la coque externe, pour produire une force d'extraction sur le fil (8) pouvant être passé à travers le tunnel (6).

6. Aide à l'extraction selon la revendication 5, **caractérisée en ce que** celle-ci présente des moyens (13, 23, 24, 31) au moyen desquels le fil passé à travers le tunnel (6) peut être serré dans l'aide à l'extraction, et en ce que celle-ci présente d'autres moyens (17, 21, 16; 29, 30, 28) par lesquels la longueur du fil (8) passée à travers le tunnel (6) peut être raccourcie.

7. Aide à l'extraction selon la revendication 6, **caractérisée en ce que** la longueur du fil à l'intérieur de l'aide à l'extraction (10) peut être raccourcie par le mouvement d'un emplacement de serrage (11), où le mouvement de l'emplacement de serrage est soutenu d'une manière cinématique à l'intérieur de l'aide à l'extraction pour éviter de grandes forces externes lors de l'actionnement de l'aide à l'extraction.
